# EUROPEAN PATENT SPECIFICATION

(11) **EP 2 445 471 B1**
(45) Date of publication and mention of the grant of the patent: **23.10.2013**
(21) Application number: 10790587.9
(22) Date of filing: 26.01.2010
(51) Int. Cl.: A61M 25/04, A61J 15/00, G06F 3/0354, G06F 3/0346

(54) **DEVICE FOR ENTERAL FEEDING**
VORRICHTUNG FÜR ENTERALE ERNÄHRUNG
DISPOSITIF D'ALIMENTATION ENTERALE

(30) Priority: 26.06.2009 IT MO20090169
(43) Date of publication of application: 02.05.2012
(73) Proprietor: De Lutio, Enrico, 05022 Amelia (TR) (IT); Danisi, Osvaldo, 80132 Napoli (IT); Azzolini, Graziano, 41032 Cavezzo (MO) (IT); Ambrosi De Magistris, Raffaele, 80132 Napoli (IT); Lettieri, Micaela, 84075 Stio (SA) (IT)
(72) Inventor: DE LUTIO, Enrico, I-05022 Amelia (IT); DANISI, Osvaldo, I-80132 Napoli (IT); AMBROSI DE MAGISTRIS, Raffaele, I-80132 Napoli (IT); LETTIERI, Micaela, I-84075 (IT)
(74) Representative: Brunacci, Marco
(86) International application number: PCT/IB2010/000131
(87) International publication number: WO 2011/018682

(56) References cited:
- EP-B- 0 824 929
- WO-A-03/092780
- WO-A-2008/027375
- WO-A1-2005/105018
- US-A- 5 073 166
- US-A- 5 112 310

## Description

### Technical Field

The present invention relates to a device for enteral feeding, particularly for the replacement of PEG probes.

### Background art

Devices for enteral feeding are medical devices used to introduce nourishing elements into the gastrointestinal tract of those patients who, though not being able or not wanting to take food through the moth, have maintained partial or total function of the digestive system.

Devices for enteral feeding can be temporary, for the treatment of acute conditions, or fixed, in the case of chronic illnesses.

US 5 073 166 A, EP 0 824 929 B, WO 2008/027375 A, US 5 112 310 A and WO 2005/105018 A1 are disclosing such devices.

A frequently used device for enteral feeding is the so-called nasal-gastric probe. This device is generally used for patients with enteral feeding programmes lasting less than one month.

In fact, although the nasal-gastric probe is easy and quick to position, besides being inexpensive, it also has a number of drawbacks.

It is, in fact, not very comfortable for the patient, who generally finds it hard to get used to the nasal probe. Furthermore, this type of device can be easily removed by coughing or vomiting. The nasal-gastric probe can also bring about the formation of areas of nasal irritation when feeding continues for a long time and finally can cause the formation of fistulae between the digestive passage and the airways.

For these reasons, in the event of enteral feeding continuing over time, for more than one month, a device is generally used called PEG (Percutaneous Endoscopic Gastrostomy) probe.

The PEG probe consists of a small tube inserted in the stomach through a tiny cut made in the abdomen.

The PEG probes, as the person skilled in the art know, can be inserted by means of the pull technique or the push technique.

The pull technique envisages that, after identifying an area of the abdomen suitable for making the cut, a needle-cannula be inserted through it to introduce a thread into the stomach, which is then captured with the endoscope and extracted through the oral cavity. To this thread is then tied the PEG probe, which is withdrawn from below until it comes out on the outside for definitive positioning.

The push technique, on the other hand, envisages the use of a guide thread and the positioning on this of the PEG probe, which is then pushed from the oral cavity until it comes out of the abdominal wall.

After a certain period of time, generally amounting to a number of months and in any case never before four weeks from the initial installation, the PEG probe is replaced at the discretion of the doctor with a less cumbersome device, i.e., a device protruding less from the abdominal wall (and therefore called low profile), suitable for upgrading the quality of the patient's life inasmuch as it does not hinder movements and provides no handholds whereby it could accidentally be removed.

The devices for enteral feeding of known type, and in particular those used to replace the PEG probes, are therefore composed of a feeding tube with an inlet mouth and with an outlet mouth, the latter to be positioned inside the patient's stomach, and also have internal and external retention means suitable for preventing the accidental removal of the tube.

More in particular, the external retention means are composed of a plaque fitting sliding on the feeding tube and which, resting on the abdominal wall, prevents the tube from moving inside the stomach by friction.

The internal retention means are on the other hand suitable for preventing the feeding tube from being accidentally removed outwards.

More in particular, the internal retention means with which the devices of known type are fitted are generally of two types, called small dome and balloon. The small dome internal retention means are in point of fact made up of an elastically deformable swelling defined in correspondence to the end part of the tube, i.e., the part comprising the outlet mouth and designed to be positioned inside the patient's stomach.

This type of internal retention means, although it has the advantage of lasting a long time, also involves a complex positioning and removal procedure.

In fact, the small dome retention means require considerable force for their extraction, due to the external overall dimensions and to the consistency of the swelling, which often causes annoyance or suffering for the patient. Furthermore, the installation and removal procedure of these small dome retention means often requires the use of an accessory instrument and the application of a certain force needed to deform the swelling itself during these phases. The deformation of the swelling, suitable for elongating it so as to reduce its overall dimensions, thus allows introducing it and extracting it from the patient's abdomen.

To relax the elastic swelling, it is therefore necessary to apply on it a constant traction force both during the introduction phase and during the extraction phase, inasmuch as such swelling tends to naturally become bigger and consequently a reduction in the force exercised on it can result in more difficult introduction or extraction. It can therefore be easily imagined just how hard it can be to apply a substantially constant force on the swelling during the entire performance of the introduction and extraction phases. Furthermore, the force applied on the swelling can, with the passing of time and therefore number of operations carried out, damage the swelling itself.

The balloon internal retention means are composed, as the term itself suggests, of a sort of inflatable balloon arranged in correspondence to the end section of the feeding tube, outside this and around the outlet mouth.

These balloons are generally inflated by means of liquid.

This type of internal retention means also has several drawbacks.

In fact, even though the devices provided with balloon internal retention means are easy to fit and replace, precisely by means of the deflation of the balloon itself, they are subject to frequent breakages or malfunctions which require their frequent replacement and, therefore, considerable disturbance for the patient, for their family members and for the medical personnel.

Furthermore, the effect of the gastric juices and the intrinsic weakness of the balloon inevitably lead to the breakage of the latter, after a period of time that varies from patient to patient. Although the breakage of the balloon does not affect the patient's health, it does however expose him/her to the risk of having to undergo a new re-implant in case the gastrostomy closes.

It must be remembered that even in the case of balloons that operate perfectly, periodical maintenance of same is nevertheless required.

Such maintenance is made necessary by the porosity of their wall which, including due to the effect of the pressure placed on it, tends to lose part of the contents. This causes the balloon to become smaller and therefore to lose its retention capacity. For this reason, every week, those who assist these patients must deflate the balloon, remove the remaining liquid and fill it again with the quantity of liquid required to bring it to maximum dilation, so as to ensure correct operation.

Furthermore, a drawback common to all types of known devices for enteral feeding, and therefore whatever the shape of the internal retention means, consists in the need to have the feeding tubes at disposal in a broad range of measurements, so as to be able to address all the requirements, both as regards their length and their gauge.

The feeding tubes of such known devices in fact are of fixed length and for this reason have to be frequently replaced inasmuch as, during the course of the patient's life, the thickness of the abdominal wall varies and the implanted system can become no longer adequate.

Hence therefore, as can be easily imagined, the need to replace the implanted devices with others of adequate dimensions that allow correct positioning.

As indicated above however, the measurements of the tubes of the devices of known type are fixed and predefined and, even though a broad range of such tubes exists, it is not always possible to find the right measurement among these. It can therefore occur that we are obliged to use the available measurements closest to the optimum measurement, which can in any case result in correct positioning.

Secondly, such variety of measurements forces hospitals to maintain stocks of all dimensions, with consequent higher costs and logistic difficulties.

### Object of the Invention

The main aim of the present invention is to provide a device for enteral feeding that can be positioned and removed easily, without causing too much disturbance to the patient and to the medical personnel, and which at the same time is distinguished by long life and safe use.

One object of the present invention is therefore to make a device for enteral feeding that can be fitted quickly and easily, which does not therefore require the exercising of a constant force for fitting and extracting the internal retention means, and which at the same time is not distinguished by a shape such as to require it to be frequently serviced and replaced.

One object of the present invention is to enable the medical personnel to choose the ideal length of feeding tube according to the patient, so as to allow the perfect positioning of the device itself.

Another object of the present invention is to reduce the need for hospital stocks with the consequent cutting of storage and management costs.

Another object of the present invention is to provide a device for enteral feeding, which allows to overcome the mentioned drawbacks of the known art in the ambit of a simple, rational, easy, effective to use and low cost solution.

The above objects are achieved by the present device for enteral feeding, according to claim 1 and its dependent claims.

### Brief Description of the Drawings

Other characteristics and advantages of the present invention will become more evident from the description of a preferred, but not sole, embodiment of a device for enteral feeding, illustrated purely as an example but not limited to the annexed drawings in which:
figure 1 is a view of the device according to the invention with the flexible elements in extended configuration;
figure 2 is a perspective view of the device of figure 1 with the flexible elements partially moved towards the widened configuration;
figure 3 is a perspective view of the device of figure 1 with the flexible elements in widened configuration;
figure 4 is a perspective view of the feeding tube of the device of figure 2; figure 5 is a perspective view of the compression element according to the invention;
figure 6 is a raised side view of a particular embodiment of the device according to the invention, having an enteral feeding catheter;

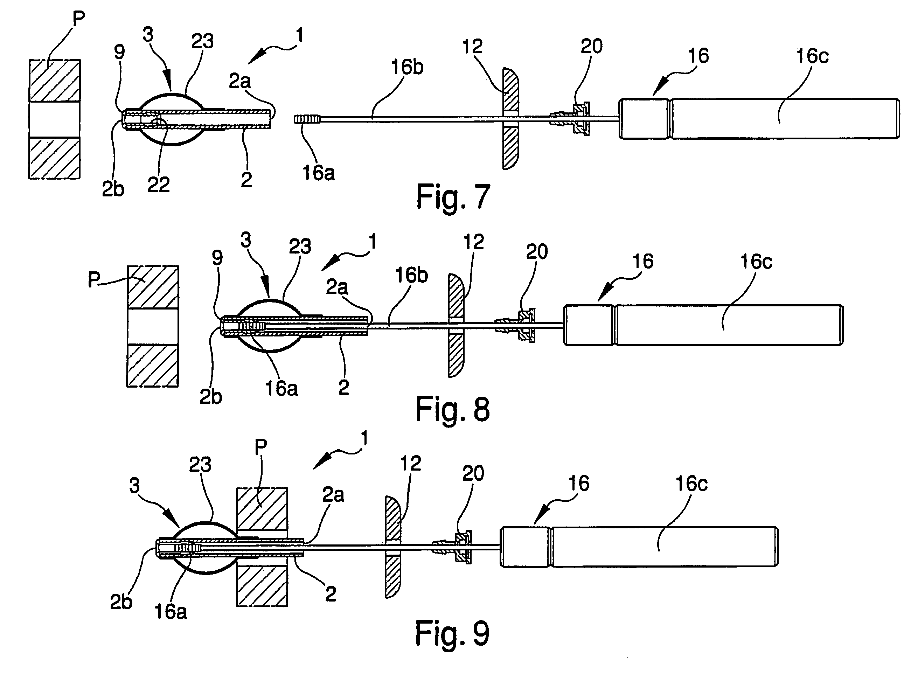

figure 7 is a raised side view of a preferred embodiment of the device according to the invention, with the relative first movement element arranged outside the feeding tube;
figure 8 is a raised side view of the device of figure 7, with the first movement element engaged with the compression element;
figure 9 is a raised side view of the device of figure 8, with the end part of the feeding tube inserted in the patient's stomach;
figure 10 is a raised side view of the device of figure 9, with the retention means in widened configuration;
figure 11 is a raised side view of the device of figure 10, with the external retention element and the connector fitted to the feeding tube;
figure 12 is a raised side view of the device according to figures from 7 to 11 applied to the wall of a patient's stomach;
figure 13 is a perspective view of an alternative embodiment of the first movement element contained in the kit according to the invention with the small arms in work position;
figure 14 is a perspective view of the device according to the invention with the first movement element of figure 13 fitted in the feeding tube;
figure 15 is a perspective view of the second movement element contained in the kit according to the invention.

### Embodiments of the Invention

With particular reference to such figures, globally indicated by 1 is a device for enteral feeding, particularly for the replacement of PEG probes.

The device 1 comprises a feeding tube 2 for administering foods, preferably made of silicone or linear polyurethane (PUR), having at least an inlet mouth 2a and at least an outlet mouth 2b and intended to be inserted at least partially inside a patient's stomach.

Preferably, as shown on the attached illustrations, on the outer surface of the tube 2 a plurality of centimetre notches are featured.

The inlet mouth 2a is associable with infusion sets for the administration of nutritional substances, not shown in the illustrations, and the outlet mouth 2b is designed to be positioned inside the patient's stomach.

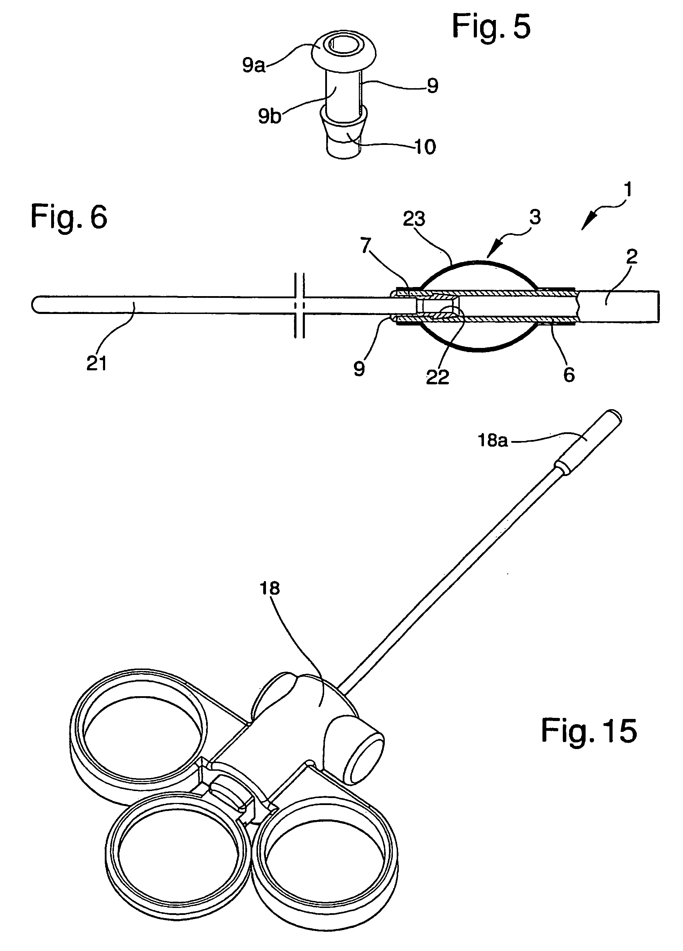

In a particular embodiment, shown in the figure 6, suitable for the administration of foods along at least a section of the small intestine (empty stomach) of a patient, the device 1 comprises an enteral feeding catheter 21 associated with the outlet mouth 2b of the tube 2. As the person skilled in the art knows, the enteral feeding catheter 21 has a suitably weighted extremity to favour positioning along the feeding tract and one or more openings, not shown in detail in the illustration, for the introduction of nutritional substances inside the small intestine.

Advantageously, the device 1 comprises a valved connector 20 associated with the inlet mouth 2a.

The connector 20 is suitably made of plastic material with rigidity greater than that of the tube 2 and is slotted into the inlet mouth 2a.

More in particular, the connector 20 has a unidirectional valve and its purpose is to prevent back-flow of gastric content towards the outside.

The connector 20 also defines a stop suitable for preventing the retention element 12 being removed from the tube 2.

The device 1 also comprises internal retention means 3 associated with the tube 2 and which can be widened to prevent accidental removal, during use, of the tube itself towards the outside.

According to the invention, the retention means 3 are mobile from an extended configuration to a widened configuration and vice versa due to the effect of the compression and of the extension respectively of at least one portion of the tube 2.

Advantageously, the retention means 3 comprise at least a flexible element 4 suitable for increasing its overall dimensions in a direction substantially crossways to the length of the tube 2 by means of the compression of at least a portion of the tube itself.

In a preferred embodiment, the device 1 comprises a plurality of flexible elements 4.

More in particular, the flexible elements 4 are defined at the lower portion of the tube 2 comprising the outlet mouth 2b intended to be positioned inside the patient's stomach.

Advantageously, the flexible elements 4 are defined in one body piece with the tube 2.

More in particular, in the embodiment shown in the figures from 1 to 4, the flexible elements 4 are defined by a corresponding number of incisions 5 that cross the entire thickness of the wall of the tube 2.

Suitably, such incisions 5 are defined longitudinally on the tube 2 and are arranged at an equal distance from each other.

The longitudinal extension of the incisions 5 is generally comprised between 1 cm and 3 cm, preferably about 2 cm.

The flexible elements 4 made this way therefore define a band of ribs branching out from a first area 6 of the tube 2 and reconnecting in correspondence to a second area 7 separated from the first area 6.

The first and the second areas 6 and 7 respectively, in point of fact define the opposite extremities of the flexible elements 4.

During the movement of the retention means 3 from the extended configuration to the widened configuration, the flexible elements 4 flex and move away from one another, defining a plurality of through openings 8 positioned between the flexible elements themselves.

Obviously, the openings 8 widen more and more as the flexible elements 4 move from extended configuration towards widened configuration. Advantageously, the device 1 then comprises compression means for compressing at least one portion of the tube 2 and, more in particular, the portion positioned between the first and the second areas 6 and 7. The compression means are therefore suitable for compressing the flexible elements 4.

More in particular, compression means comprise at least a compression element 9 having greater rigidity than that of the feeding tube 2.

In the embodiment shown in figure 5, the compression element 9 has a swelling 9a defined in correspondence to an extremity of the stem 9b suitable for insertion inside the tube 2 through the outlet mouth 2b.

More in particular, the compression element 9 has a tubular shape and is therefore open at its extremities to allow the transit of the nutritional substances and is inserted with the stem 9b inside the tube 2 through the outlet mouth 2b. The insertion of the compression element 9 in the tube 2 terminates due to the stop of the swelling 9a against the bottom wall of the tube 2 which defines the outlet mouth 2b.

The compression element 9 is suitably mobile between an idle configuration, in which it is moved away from the inlet mouth 2a and the flexible elements 4 are in extended configuration, and an active configuration, in which it is moved close to the inlet mouth 2a with respect to the idle configuration and the flexible elements 4 are in widened configuration, i.e., they are bent in such a way as to increase their overall dimensions crossways to the length of the tube 2.

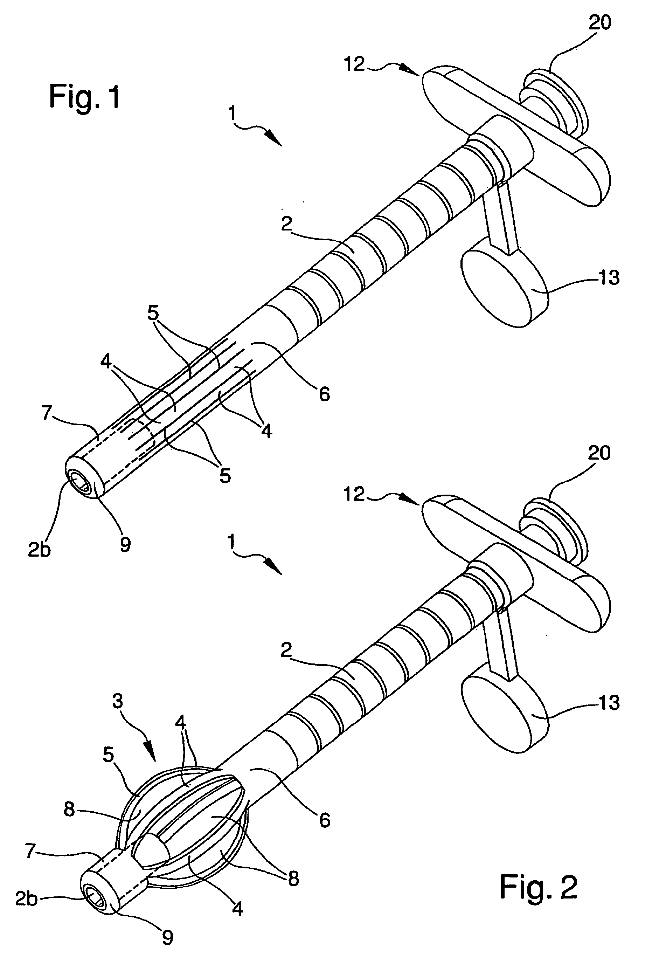

The movement of the compression element 9 from the idle configuration to the active configuration thus requires the corresponding movement of the flexible elements 4 from the extended configuration to the widened configuration. More in particular, in the idle configuration shown in figure 1, the extremity of the stem 9b opposite the swelling 9a is arranged in correspondence to the second area 7 and is separated from the first area 6.

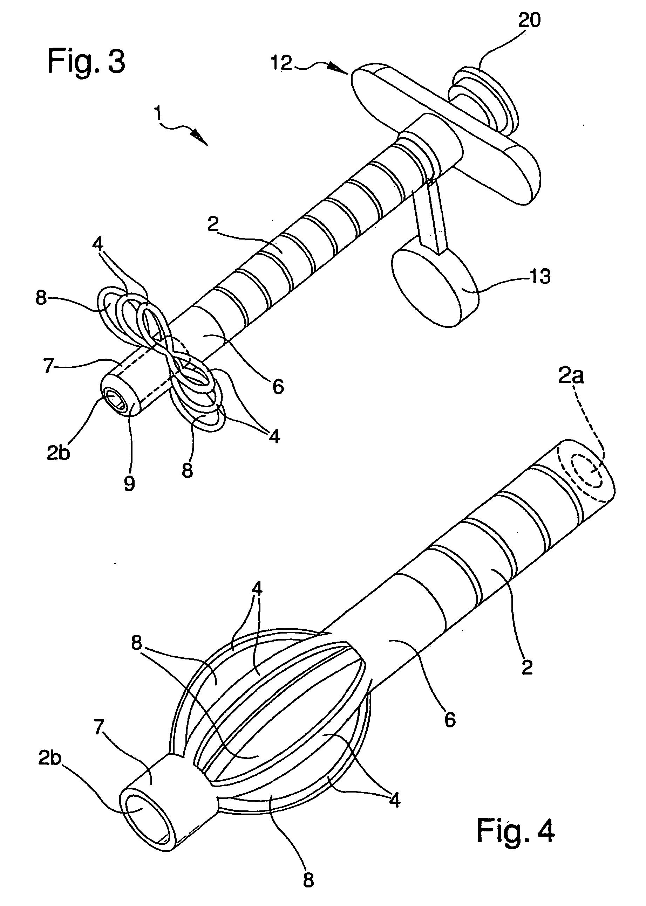

In the active configuration shown in figure 4, the extremity of the stem 9b opposite the swelling 9a is on the other hand fitted inside the tube 2 in correspondence to the first area 6, in such a way as to define a duct without discontinuity for the transit of the nutritional substances.

In this configuration therefore, the second area 7 is moved close to the first area 6.

The incisions 5 give greater compliance to the portion of tube 2 between the first and the second areas 6 and 7, allowing it to deform easier compared to the remaining parts. At the same time, the material of which the tube 2 is made and the thickness of its wall give sturdiness to the flexible elements 4.

Suitably, the device 1 comprises removable blocking means for blocking the compression element 9 in the active configuration.

More in particular, the blocking means comprise one or more protrusions 10 defined on the outer surface of the stem 9b to block the compression element 9 with respect to the first area 6 of the tube 2.

In point of fact, the protrusion 10 defines a stop suitable for engaging, when the compression element 9 is fitted inside the first area 6, against a relative counter-stop defined by the inner surface of the tube 2, in such a way that the compression element 9 slots into the tube itself, in particular into its first area 6. Advantageously, the compression element 9 has engagement means 22 with at least a first movement element 16,116 suitable for bringing the retention means 3 at least from extended configuration to widened configuration.

More in particular, as shown in the figures from 7 to 11, the engagement means 22 comprise a first thread suitable for engaging with a second thread 16a defined on the first movement element 16.

Preferably, the first thread 22 is an inner thread, i.e., it is defined on the inner wall of the compression element 9, and the second thread 16a is defined on the outer surface of the extremity section of the first movement element 16. Advantageously, the retention means 3 also comprise a covering film 23, which can be seen in detail in figures from 6 to 12, associated externally with the tube 2 in correspondence to the flexible elements 4.

Such covering film 23 defines in point of fact a substantially continuous outer surface suitable for avoiding the formation of encrustations in the openings 8 defined between the various flexible elements 4, which would prevent their return to the extended configuration.

Preferably, but nor exclusively, the covering film 23 is made of the same material as the tube 2.

In widened configuration, therefore, the flexible elements 4 are bent to form a sort of "onion" intended to be rested against the inner part of the patient's abdominal wall P to prevent the accidental removal of the device 1.

The device 1 then also comprises at least an external retention element 12 associated with the tube 2 and intended to be rested against the outer part of the patient's abdominal wall P.

The retention element 12, preferably made of silicone, comprises an opening suitable for being fitted on the tube 2 and a cover 13 for closing this opening.

The present invention also relates to a kit for enteral feeding, comprising at least a device 1 according to the above description and at least a first movement element 16,116 that can be fitted inside the tube 2 to bring the retention means 3 of the device 1 at least from extended configuration to widened configuration. More in particular, the first movement element 16,116 is suitable for engaging with the compression element 9 to move it at least from the idle configuration to the active configuration.

As mentioned above, in the preferred embodiment shown in the figures from 7 to 11, the first movement element 16 has a second thread 16a defined on the outer surface of its extremity section and suitable for engaging, as shown in the figures from 8 to 11, with the first thread 22 defined on the compression element 9. In this embodiment, the first movement element 16 is suitable for moving the compression element 9 both from the idle configuration to the active configuration and vice versa.

More in particular, the first movement element 16 shown in the figures from 7 to 11, comprises a body 16b having the second thread 16a at one extremity and a grip 16c at the opposite extremity.

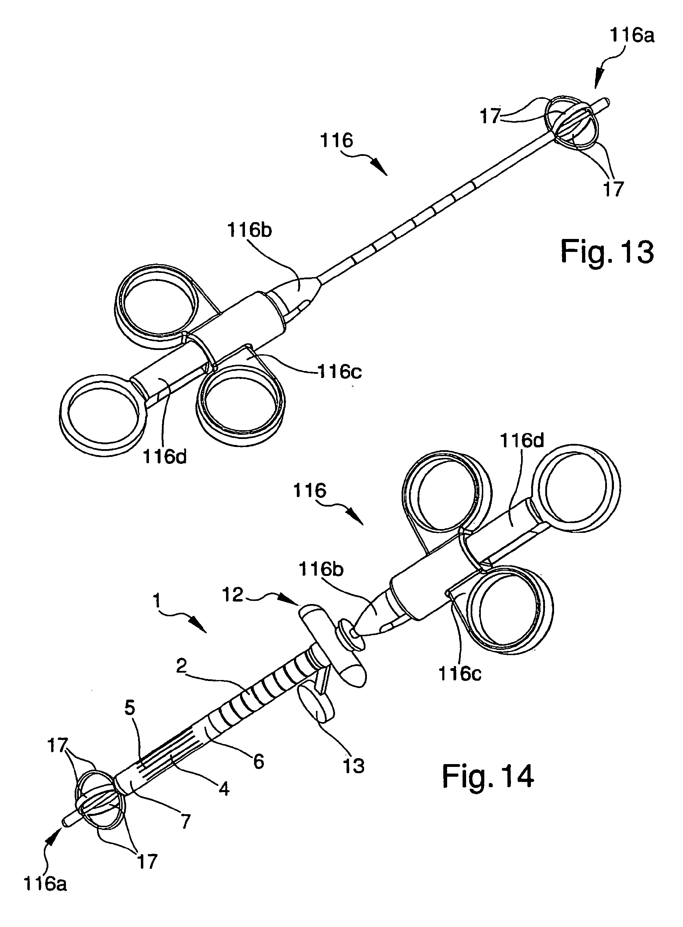

In an alternative embodiment, shown in the figures 13 and 14, the first movement element 116 comprises an extremity 116a intended, during use, to exit from the outlet mouth 2b and which can be widened to engage with the compression element 9 associated with the tube 2.

More in particular, the first movement element 116, also called "delivery system or holder", has a body 116b on which are obtained a series of centimetre notches, a grip 116c with rings and a piston 116d connected to the extremity 116a and inserted sliding inside the grip 116c.

The extremity 116a has a plurality of longitudinal incisions, not visible in detail in the illustrations, which define a corresponding number of small bending arms 17. The small arms 17, in the same way as described for the flexible elements 4, are mobile between an idle position, in which they are substantially extended, and a work position, in which they are bent in such a way as to increase their overall external dimensions.

The movement of the small arms 17 from the idle position to the work position and vice versa is done by operating the piston 116d, which produces a traction action on the extremity 116a which results in the flexion and therefore the widening of the small arms 17.

Advantageously, inside the kit according to the invention, the first movement element 16,116 is fitted inside the tube 2, on which is pre-fitted at least the external retention element 12 as shown in figure 14.

More in particular, the first instrument 116 is inserted, with the small arms 17 in idle position, inside the tube 2 starting from the inlet mouth 2a, it passes through the retention element 12 and exits with its extremity 116a from the outlet mouth 2b.

In a first embodiment of the kit according to the invention, the latter comprises, besides the device 1 described above, also at least a first movement element 16, which allows moving the compression element 9 from the idle configuration to the active configuration and vice versa.

In a second embodiment, the kit according to the invention comprises, besides the device 1 described above, the first movement element 116 suitable for moving the retention means 3 from the extended configuration to the widened configuration and also a second movement element 18 suitable for returning the retention means 3 to the extended configuration. Preferably, the second movement element 18, shown in figure 15, is arranged inside the kit, separated from the other elements. More in particular, the second movement element 18 can be inserted inside the tube 2 and has an extremity 18a intended to engage with the compression element 9 to disengage it from the first area 6 of the tube 2 by applying a force in the direction of moving away from the inlet mouth 2a, so as to return it to the idle configuration.

Again, the kit according to the invention can also comprise cutting means for cutting the tube 2. More in particular, the cutting means comprise shears having a recess for housing the tube 2.

Such shears, not shown in the illustrations, are used to shear the tube 2 at the required length, after measuring the thickness of the gastrostomy.

Preferably, the kit according to the invention also comprises lubricating means, e.g., a gel, applicable to the device 1. In particular, the gel is applied on the tube 2 for easier insertion inside the stomy.

In an alternative embodiment of the kit according to the invention, the various elements making it up, and in particular the elements making up the device 1, can be arranged separated the one from the other, in such a way that the operator then sees to assembly.

The operation of the present invention is as follows.

The kit is expected to be used for enteral feeding in the two embodiments described above.

The patient is also expected to already have a gastrostomy on the abdomen created by the insertion of a PEG probe and the latter is expected to already have been removed.

First of all, the length of the gastrostomy is measured by means of an L-shaped measuring device, not shown in the illustrations, or by means of the centimetre notches shown on the first movement element 16,116.

The first movement element 116 is then therefore inserted, e.g., inside the gastrostomy and the length of the latter is measured thanks to the centimetre notches with which it is equipped and which start immediately above the small arms 17 and end close to the grip 116c.

After taking the gastrostomy measurement the tube 2 is cut by means of the shears to the required length using the centimetre notches with which it too is equipped.

Obviously, cutting the tube 2 requires the definition of a new inlet mouth 2a. At this point the first movement element 16,116 is fitted in the tube 2, and the whole is fitted through the gastrostomy, so as to move the end part of the tube 2 comprising the outlet mouth 2b inside the patient's stomach.

In the preferred embodiment of the kit according to the invention, i.e., comprising the first movement element 16, before fitting the tube 2 inside the patient's stomach, the first movement element itself is engaged with the compression element 9, i.e., the second thread 16a is screwed up to the first thread 22. This way the first movement element 16 and the compression element 9 are integral the one with the other.

Suitably, before fitting the first movement element 16 inside the tube 2, its body 16b is made to pass through the external retention element 12 and the connector 20.

After fitting the tube 2 inside the patient's stomach, a direct traction force is exercised towards the outside of the stomach, at the same time blocking the tube 2, through the first movement element 16. Such traction force causes the movement of the compression element 9, integral with the first movement element 16, towards the first area 6 of the tube 2, i.e., towards its active configuration.

By effect of such force exercised on the compression element 9, the second area 7 is brought nearer to the first area 6, bending the flexible elements 4. The traction force on the compression element 9 is exercised until, moving to the active configuration, it fits itself inside the first area 6 and its protrusions 10 engage with the corresponding protrusions defined on the inner wall of the tube 2.

More in particular, in this configuration, the flexible elements 4 are bent by the reciprocal approach of the two extremities thereof, represented by the first and the second area 6 and 7 of the tube 2, in actual fact defining a swelling outside the tube itself.

After moving the compression element 9 to the active configuration, and therefore the retention means 3 to the widened configuration, the external retention element 12 is moved into contact with the patient's stomach, making it slide with respect to the body 16b and fitting it on the extremity part of the tube 2, after which, the connector 20 is fitted inside the inlet mouth 2a.

Different embodiments of the procedure according to the invention cannot however be ruled out, which envisage the application of the retention element 12 and the connector 20 to the tube 2 immediately after having cut the tube itself at the required length and before fitting the first movement element 16, or which envisage the cut of the tube 2 after positioning the retention element 12 resting on the external part of the abdominal wall P.

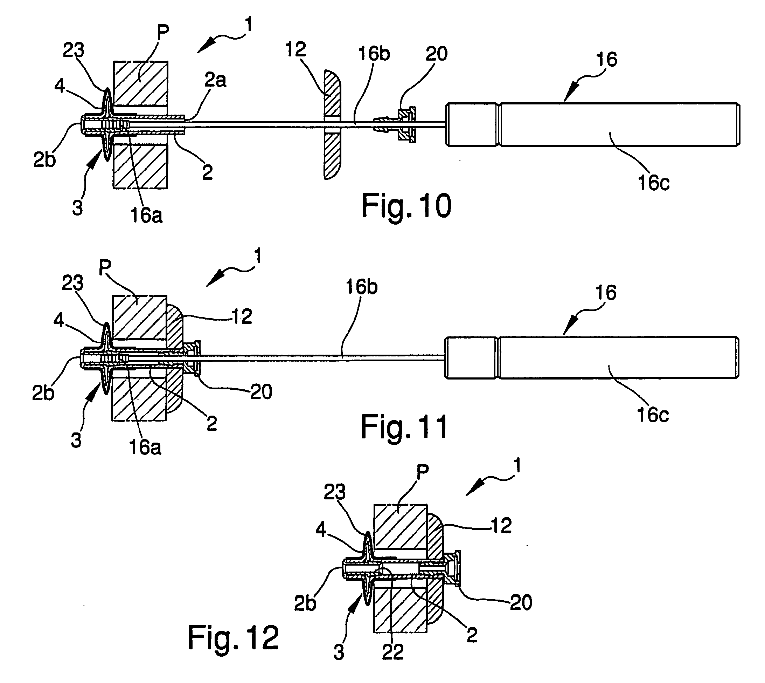

After having applied both the retention element 12 and the connector 20 to the tube 2, the first movement element 16 is disengaged from the compression element 9, unscrewing it as shown in figure 11, and the first movement element is removed from the tube 2 making it pass through the retention element 12 and the connector 20.

This way, the device 1 appears correctly fitted, as shown in the figure 12, and at the same time has the least possible external overall dimensions.

The device 1 is therefore ready to use, and it is therefore possible to connect an infusion set for the administration of the nutritional substances to the connector 20 placed in the inlet mouth 2a of the tube 2 defined by the cutting of the latter. In the event of having to remove the device 1 from the patient, the retention means 3 will have to be returned to extended configuration, so as to then be able to pull the tube 2 out towards the outside.

This operation is performed by again engaging the first movement element 16 with the compression element 9, screwing it onto this in order to make them integral the one with the other. At this point, the first movement element 16 is pushed towards the patient, i.e., in a moving away direction from the inlet mouth 2a, so as to move the compression element 9 away from the first area 6. The force applied to the compression element 9 must be such as to allow the disengagement of its protrusion 10 from the tube 2.

After having returned the compression element 9 to its idle configuration, and therefore the retention means 3 to the extended configuration, the device 1 is removed from the patient's stomach by pulling the first movement element 16 outwards, the latter being still engaged with the compression element 9.

In the alternative embodiment of the kit according to the invention, i.e., comprising the first movement element 116, the first movement element 116 is only operated after fitting the tube 2 inside the patient's stomach. In this case too, however, before bringing its extremity 116a inside the tube 2, its body 116b is fitted through the external retention element 12 and the connector 20.

The operation of the first movement element 116 causes the movement of the piston 116d and the consequent widening of the extremity 116a that exits from the outlet mouth 2b as a result of the bending of the small arms 17, which move to the work position.

The extremity 116a, in widening, defines a stop designed to rest against the compression element 9 associated with the outlet mouth 2b.

By therefore applying a traction force on the first instrument 116 directed towards the outside, the extremity 116a applies a consequent action on the compression element 9, which from the idle configuration is pushed towards the inlet mouth 2a, thus compressing the portion of tube 2 placed between the first and the second area 6 and 7.

Similarly to what has been described above for the preferred embodiment, the second area 7 is moved closer to the first area 6, by effect of the force applied on the compression element 9, blending the flexible elements 4. The traction force on the compression element 9 is applied until this, by moving to the active configuration, fits inside the first area 6 and its protrusions 10 engage with the corresponding protrusions defined on the inner wall of the tube 2. As described above, the movement of the compression element 9 to the active configuration produces the movement of the retention means 3 to the widened configuration. The flexible elements 4 in widened configuration prevent the tube 2 from being accidentally extracted outwards or in any case moved from the correct operating position, and consequently restrain the position inside the patient's stomach. The compression element 9 being therefore blocked in the active configuration, the first instrument 116 can now be removed

Similarly to what has been described above, in this case as well, before removing the first movement element 116, the external retention element 12 is fitted on the tube 2, bringing this into contact with the patient's stomach, and the connector 20 is fitted inside the inlet mouth 2a.

Different embodiments of the procedure according to the invention cannot however be ruled out envisaging the fitting of the retention element 12 and of the connector 20 to the tube 2 immediately after having cut the tube itself at the required length and before inserting the first movement element 116, or envisaging the cutting of the tube 2 after the positioning of the retention element 12 resting on the external part of the abdominal wall P of the patient.

To remove the first instrument 116 the relevant piston 116d must again be operated in the direction opposite to the previous one, in such a way as to return the small arms 17 to idle position, thereby extending the extremity 116a.

The first movement element 116 can therefore be extracted from the tube 2 through the connector 20 fitted in the inlet mouth 2a.

This way, the device 1 is correctly positioned and at the same time has the least possible external overall dimensions.

The device 1 is therefore ready to use, and an infusion set can be connected for the administration of nutritional substances to the connector 20 placed in the inlet mouth 2a of the tube 2 defined by the cutting of the latter.

As mentioned above for the first movement element 16, in the event of having to remove the device 1 from the patient, the retention means 3 will have to be returned to extended configuration so to then be able to extract the tube 2 outwards.

To perform this operation, the second movement element 18 is inserted inside the tube 2, passing through the connector 20, until it contacts the compression element 9 with its extremity 18a.

After bringing the extremity of the second movement element 18 in contact with the compression element 9, a direct force is applied on the latter in the direction of moving away from the inlet mouth 2a. This force must be enough to disengage the protrusions 10 from the tube 2.

The compression element 9 is then returned to the idle configuration and, consequently, the second area 7 is moved away from the first area 6.

The reciprocal moving away of the areas 6 and 7 of the tube 2 causes the extension of the flexible elements 4, which again reduce their overall dimensions in a direction crossways to the length of the tube itself.

The tube can now therefore be easily removed from the patient's abdomen.

In both the described embodiments, the fixing and the removing of the device 1 are therefore performed, respectively, by means of the compression and extension of the portion of the tube 2 placed between the first and the second area 6 and 7, i.e., by moving the extremities of the flexible elements 4 which make up the internal retention means 3 closer together or away from one another.

The deformation of the flexible elements 4 occurs by simply acting on the compression element 9, i.e., by moving it alternately from the idle configuration to the active configuration and vice versa.

Advantageously, in the event of having to make a first installation, the device 1 according to the invention can be applied using the known method, i.e., by inserting a traction thread through the gastrostomy defined on the patient's abdominal wall, intercepting this thread by means of a gastroscope in such a way as to be able to extract it from the patient's mouth and then associating the tube 2 at the extremity of this thread, so as to be able to recall the latter by effect of the traction exercised on the thread itself from outside. This way, the tube 2 is made to pass inside the stomach through the abdominal wall. The fixing of the tube to the patient's abdominal wall then occurs according to the same procedures described above.

It has in point of fact been ascertained how the described invention achieves the proposed objects and in particular the fact is underlined that the device for enteral feeding according to the invention allows overcoming the drawbacks of the state of the art, and is easy to fit and remove and at the same time safe to use and long lasting.

In fact, the particular shape of the flexible elements and the fact that these are defined integrally with the feeding tube makes them very sturdy and prevents their accidental breakage, and at the same time allows their easy deformation. Again the device according to the invention allows modifying the configuration of the internal retention means before these are fitted or removed and maintaining such configuration without having to apply any force on them. Furthermore, the kit for enteral feeding according to the invention allows adapting the device for enteral feeding to each individual patient, permitting ideal positioning whatever the physical build of the patient him/herself.

The device for enteral feeding according to the invention can therefore be implanted in and removed from a patient in a non-traumatic and fast way; furthermore, its particular structure allows it to be used for a long time inasmuch as it is very unlikely to be affected by breakages or malfunctions.

## Claims

1. Device (1) for enteral feeding, comprising:
- a feeding tube (2) for administering a nutritional substance having at least an inlet mouth (2a) and at least an outlet mouth (2b) and which can be inserted at least partially inside the stomach of a patient;
- internal retention means (3) associated with said tube (2) and which can be widened to prevent the accidental removal, during use, of said feeding tube (2) towards the outside of the stomach;
wherein said retention means (3) are mobile from an extended configuration to a widened configuration and vice versa by means of the compression and the extension respectively of at least a portion of said tube (2);
wherein the retention means (3) comprise a plurality of flexible elements (4), defmed in one body piece with said tube (2) in correspondence to the lower portion thereof comprising said outlet mouth (2b), by means of a corresponding number of incisions (5) crossing the entire thickness of a wall of the tube (2) so that the flexible elements (4) defines a band of ribs branching from a first area (6) of the tube (2) and reconnecting in correspondence to a second area (7) of the tube (2), separated from the first area (6) and comprising the outlet mouth (2b), which flexible elements (4) are suitable for increasing their overall dimensions, crossways to said tube (2), by means of their compression;
the device (1) comprising compression means for compressing the flexible elements (4) and being **characterized in that**:
the compression means comprise a tubular shaped compression element (9) open at its extremities to allow the transit of said nutritional substances, having greater rigidity than that of the feeding tube (2), and further having a stem (9b) inserted inside the tube (2), which stem (9b) has a swelling (9a) defined in correspondence to an extremity which is placed against the bottom wall of the tube (2) which defines the outlet mouth (2b); said compression element (9) being movable between an idle configuration, in which it is away from said inlet mouth (2a), and the retention means (3) are in the extended configuration, and an active configuration, in which it is close to the inlet mouth (2a), with respect to the idle configuration, and the retention means (3) are in the widened configuration, and in which the flexible elements (4) are bent so as to increase their overall dimensions crossways to the length of the tube (2) and an extremity of the stem (9b) opposite to said swelling (9a) is fitted inside the tube (2) in correspondence to said first area (6) so as to define a duct without discontinuity for the transit of the nutritional substance; and **in that**
removable blocking means are comprised in the device (1) for blocking said compression element (9) in said active configuration, in turn comprising at least a protrusion (10) defined on the outer surface of the compression element (9) for engaging a corresponding protrusion defined in the inner surface of the tube (2) in said active configuration.

2. Device (1) according to claim 1, **characterized by** the fact that said incisions (5) are defined longitudinally on said tube (2).

3. Device (1) according to claim 1, **characterized by** the fact that said incisions (5) have a length ranging from 1 to 3 cm.

4. Device (1) according to claim 1, **characterized by** the fact that said compression element (9) comprises engagement means (22) with at least a first movement element (16, 116) suitable for bringing said retention means (3) at least from extended configuration to widened configuration.

5. Device (1) according to claim 4, **characterized by** the fact that said engagement means (22) comprise at least a first thread which can be engaged with a second thread (16a) defined on said first movement element (16).

6. Device (1) according to claim 5, **characterized by** the fact that said first thread (22) is defined on the inner surface of said compression element (9).

7. Device (1) according to one or more of the preceding claims , **characterized by** the fact that said retention means (3) comprise at least a covering film (23) associated externally with said tube (2) at least in correspondence to said flexible elements (4).

8. Device (1) according to claim 7, **characterized by** the fact that said covering film (23) is made of the same material of said tube (2).

9. Device (1) according to one or more of the preceding claims, **characterized by** the fact that it comprises at least an external retention element (12) associated with said tube (2).

10. Device (1) according to one or more of the preceding claims, **characterized by** the fact that it comprises at least an enteral feeding catheter (21) associated with the outlet mouth (2b) of said tube (2).

11. Kit for enteral feeding, **characterized by** the fact that it comprises at least a device (1) for enteral feeding according to one or more of the claims from 1 to 10 and at least a first movement element (16, 116) that can be fitted in the feeding tube (2) of said device (1) to bring the retention means (3) of the device itself at least to widened configuration.

12. Kit according to claim 11, **characterized by** the fact that said device (1) comprises at least a compression element (9) according to claim 5 or 6 and by the fact that said first movement element (16) comprises a second thread (16a), suitable for engaging with the first thread (2b) of said compression element (9).

13. Kit according to claim 11, **characterized by** the fact that said device (1) comprises at least a compression element (9) according to claim 1 and by the fact that said first movement element (116) comprises an extremity (116a), intended to exit from the outlet mouth (2b) of said tube (2) and which can be widened to engage with said compression element (9).

14. Kit according to claim 13, **characterized by** the fact that it comprises at least a second movement element (18) that can be inserted in said tube (2) to bring said retention means (3) to extended configuration.

15. Kit according to claim 13 or 14, **characterized by** the fact that said device (1) comprises at least a compression element (9) according to the claim 1 and by the fact that said second movement element (18) comprises an extremity (18a), intended to engage with said compression element (9).

16. Kit according to one or more of the claims from 12 to 15, **characterized by** the fact that it comprises cutting means for cutting said tube (2).

## Patentansprüche

1. Vorrichtung (1) zur enteralen Ernährung, enthaltend:
- einen Ernährungsschlauch (2) zur Verabreichung eines Nährstoffes, welcher mindestens eine Eingangsöffnung (2a) und mindestens eine Ausgangsöffnung (2b) aufweist und der zumindest teilweise in das Innere des Magens eines Patienten eingeführt werden kann;
- eine mit dem Schlauch (2) verbundene interne Rückhalteeinrichtung (3), die aufgeweitet werden kann, um während der Verwendung die unbeabsichtigte Entfernung des Ernährungsschlauchs (2) nach außerhalb des Magens zu verhindern;
wobei die Rückhalteeinrichtung (3) aus einer ausgestreckten Konfiguration in eine aufgeweitete Konfiguration und umgekehrt mittels der Kompression bzw. der Aufweitung zumindest eines Teils des Schlauches (2) beweglich ist;
wobei die Rückhalteeinrichtung (3) eine Vielzahl von flexiblen Elementen (4) umfasst, die einstückig mit dem Schlauch (2) seinem die Ausgangsöffnung (2b) aufweisenden unteren Abschnitt entsprechend gebildet sind, und zwar durch eine entsprechende Anzahl von Einschnitten (5), welche die gesamte Dicke einer Wand des Schlauches (2) durchqueren, so dass die flexiblen Elemente (4) ein Band aus Rippen bilden, die sich von einem ersten Bereich (6) des Schlauches (2) verzweigen und einem zweiten Bereich (7) des Schlauches (2) entsprechend wieder vereinen, der von dem ersten Bereich (6) getrennt ist und die Ausgangsöffnung (2b) aufweist, welche flexiblen Elemente (4) geeignet sind, ihre Gesamtabmessungen in Querrichtung zu dem Schlauch (2) mittels ihrer Kompression zu vergrößern;
wobei die Vorrichtung (1) eine Kompressionseinrichtung zum Komprimieren der flexiblen Elemente (4) aufweist und **dadurch gekennzeichnet ist, dass**:
die Kompressionseinrichtung ein rohrförmiges Kompressionselement (9) aufweist, welches an seinen Enden offen ist, um den Durchgang der Nährstoffe zu erlauben, eine größere Steifigkeit als die des Ernährungsschlauchs (2) aufweist und ferner einen in den Schlauch (2) eingeführten Schaft (9b) aufweist, welcher Schaft (9b) eine Verdickung (9a) hat, die einem Ende entsprechend gebildet ist, welches gegen die untere Wand des Schlauches (2) platziert wird, welche die Ausgangsöffnung (2b) bildet; wobei das Kompressionselement (9) zwischen einer Ruhestellung, in welcher es von der Eingangsöffnung (2a) entfernt ist und die Rückhalteeinrichtung (3) in der ausgestreckten Konfiguration ist, und einer aktiven Stellung beweglich ist, in welcher es in Bezug auf die Ruhestellung nahe an der Ausgangsöffnung (2a) ist und die Rückhalteeinrichtung in der aufgeweiteten Konfiguration ist und in welcher die flexiblen Elemente (4) so gebogen sind, dass sie die Gesamtabmessungen in Querrichtung zur Länge des Schlauches (2) erhöhen und ein der Verdickung (9a) entgegengesetztes Ende des Schafts (9b) dem ersten Bereich (6) entsprechend in den Schlauch (2) eingesetzt ist, um so eine ununterbrochene Leitung für den Durchtritt der Nährstoffe zu bilden; und dadurch, dass
in der Vorrichtung (1) eine entfernbare Blockiereinrichtung enthalten ist, um das Kompressionselement (9) in der aktiven Stellung zu blockieren, welche wiederum mindestens einen auf der äußeren Oberfläche des Kompressionselements (9) gebildeten Vorsprung (10) für den Eingriff in der aktiven Konfiguration mit einem entsprechenden Vorsprung aufweist, der in der Innenfläche des Schlauches (2) gebildet ist.

2. Vorrichtung (1) nach Anspruch 1, **gekennzeichnet durch** die Tatsache, dass die Einschnitte (5) in Längsrichtung an dem Schlauch (2) gebildet sind.

3. Vorrichtung (1) nach Anspruch 1, **gekennzeichnet durch** die Tatsache, dass die Einschnitte (5) eine Länge im Bereich von 1 bis 3 cm haben.

4. Vorrichtung (1) nach Anspruch 1, **gekennzeichnet durch** die Tatsache, dass das Kompressionselement (9) eine Eingriffseinrichtung (22) mit mindestens einem ersten Bewegungselement (16, 116), welches dafür geeignet ist, die Rückhalteeinrichtung (3) zumindest aus der ausgestreckten Konfiguration in die aufgeweitete Konfiguration zu bringen.

5. Vorrichtung (1) nach Anspruch 4, **gekennzeichnet durch** die Tatsache, dass die Eingriffseinrichtung (22) mindestens ein erstes Gewinde aufweist, welches mit einem zweiten Gewinde (16a) in Eingriff gebracht werden kann, welches an dem ersten Bewegungselement (16) gebildet ist.

6. Vorrichtung (1) nach Anspruch 5, **gekennzeichnet durch** die Tatsache, dass das erste Gewinde (23) an der Innenfläche des Kompressionselements (9) gebildet ist.

7. Vorrichtung (1) nach einem oder mehreren der vorhergehenden Ansprüche, **gekennzeichnet durch** die Tatsache, dass die Rückhalteeinrichtung (3) mindestens eine Abdeckfolie (23) aufweist, die äußerlich mit dem Schlauch (2) zumindest den flexiblen Elementen (4) entsprechend verbunden ist.

8. Vorrichtung (1) nach Anspruch 7, **gekennzeichnet durch** die Tatsache, dass die Abdeckfolie (23) aus demselben Material wie der Schlauch (2) hergestellt ist.

9. Vorrichtung (1) nach einem oder mehreren der vorhergehenden Ansprüche, **gekennzeichnet durch** die Tatsache, dass sie mindestens ein externes Rückhalteelement (12) aufweist, das mit dem Schlauch (2) verbunden ist.

10. Vorrichtung (1) nach einem oder mehreren der vorhergehenden Ansprüche, **gekennzeichnet durch** die Tatsache, dass sie mindestens einen Katheter (21) zur enteralen Ernährung aufweist, der mit der Ausgangsöffnung (2b) des Schlauches (2) verbunden ist.

11. Kit zur enteralen Ernährung, **gekennzeichnet durch** die Tatsache, dass es mindestens eine Vorrichtung (1) zur enteralen Ernährung gemäß einem oder mehreren der Ansprüche von 1 bis 10 und mindestens ein erstes Bewegungselement (16, 116) aufweist, welches in den Ernährungsschlauch (2) der Vorrichtung (1) eingesetzt werden kann, um die Rückhalteeinrichtung (3) der Vorrichtung selbst zumindest in die aufgeweitete Konfiguration zu bringen.

12. Kit nach Anspruch 11, **gekennzeichnet durch** die Tatsache, dass die Vorrichtung (1) mindestens ein Kompressionselement (9) nach Anspruch 5 oder 6 aufweist, und **durch** die Tatsache, dass das erste Bewegungselement (16) ein zweites Gewinde (16a) aufweist, welches für den Eingriff mit dem ersten Gewinde (2b) des Kompressionselements (9) geeignet ist.

13. Kit nach Anspruch 11, **gekennzeichnet durch** die Tatsache, dass die Vorrichtung (1) mindestens ein Kompressionselement (9) nach Anspruch 1 aufweist, und **durch** die Tatsache, dass das erste Bewegungselement (116) ein Ende (116a) aufweist, welches dafür vorgesehen ist, aus der Ausgangsöffnung (2b) des Schlauches (2) auszutreten, und welches für den Eingriff mit dem Kompressionselement (9) erweitert werden kann.

14. Kit nach Anspruch 13, **gekennzeichnet durch** die Tatsache, dass es mindestens ein zweites Bewegungselement (18) aufweist, welches in den Schlauch (2) eingeführt werden kann, um die Rückhalteeinrichtung (3) in die ausgestreckte Stellung zu bringen.

15. Kit nach Anspruch 13 oder 14, **gekennzeichnet durch** die Tatsache, dass die Vorrichtung (1) mindestens ein Kompressionselement (9) nach Anspruch 1 aufweist, und **durch** die Tatsache, dass das zweite Bewegungselement (18) einen Ende (18a) aufweist, welches für den Eingriff mit dem Kompressionselement (9) vorgesehen ist.

16. Kit nach einem oder mehreren der Ansprüche von 12 bis 15, **gekennzeichnet durch** die Tatsache, dass es eine Schneideinrichtung zum Schneiden des Schlauches (2) aufweist.

## Revendications

1. Dispositif (1) d'alimentation entérale, comprenant :
- un tube d'alimentation (2) pour administrer une substance nutritionnelle, ayant au moins un orifice d'entrée (2a) et au moins un orifice de sortie (2b) et qui peut être inséré au moins partiellement à l'intérieur de l'estomac d'un patient ;
- des moyens de retenue (3) associés audit tube (2) et qui peuvent être élargis pour empêcher le retrait accidentel, pendant l'utilisation, dudit tube d'alimentation (2) hors de l'estamac ;
dans lequel lesdits moyens de retenue (3) sont mobiles d'une configuration allongée à une configuration élargie, et vice versa, par la compression et l'extension respectives d'au moins une partie dudit tube (2) ;
dans lequel lesdits moyens de retenue (3) comprennent une pluralité d'éléments flexibles (4) définis d'une seule pièce avec ledit tube (2), en correspondance avec la portion inférieure de celui-ci comprenant ledit orifice de sortie (2b), au moyen d'un nombre correspondant d'incisions (5) traversant la totalité de l'épaisseur d'une paroi du tube (2) de façon que les éléments flexibles (4) définissent une bande de nervures se séparant depuis une première zone (6) du tube (2) et se réunissant à nouveau au niveau d'une deuxième zone (7) du tube (2), séparée de la première zone (6) et comprenant l'orifice de sortie (2b), ces éléments flexibles (4) étant aptes à accroître leurs dimensions d'ensemble, transversalement audit tube (2), par leur compression ;
le dispositif (1) comprenant des moyens de compression pour comprimer les éléments flexibles (4) et étant ***caractérisé en ce que:***
les moyens de compression comprennent un élément de compression de forme tubulaire (9) ouvert à ses extrémités pour permettre le passage desdites substances nutritionnelles, élément ayant une rigidité supérieure à celle du tube d'alimentation (2), et comprenant en outre une tige (9b) insérée à l'intérieur du tube (2), cette tige (9b) présentant un renflement (9a) défini en correspondance d'une extrémité qui est placée contre la paroi de fond du tube (2) qui définit l'orifice de sortie (2b) ; ledit élément de compression (9) étant mobile entre une configuration de repos, dans laquelle il est éloigné dudit orifice d'entrée (2a) et les moyens de retenue (3) sont dans la configuration allongée, et une configuration active, dans laquelle il est proche de l'orifice d'entrée (2a), par rapport à la configuration de repos, et les moyens de retenue (3) sont dans la configuration élargie, et dans laquelle les éléments flexibles (4) sont incurvés afin d'augmenter leurs dimensions d'ensemble transversalement à la longueur du tube (2) et une extrémité de la tige (9b) opposée audit renflement (9a) est placée à l'intérieur du tube (2) en correspondance avec ladite première zone (6) afin de définir un conduit sans discontinuité pour le passage de la substance nutritionnelle *; **et en ce que***
des moyens de blocage amovibles sont compris dans le dispositif (1) pour bloquer ledit élément de compression (9) dans ladite configuration active, à leur tour comprenant au moins une saillie (10) définie sur la surface extérieure de l'élément de compression (9) pour engager une saillie correspondante définie dans la surface intérieure du tube (2) dans ladite configuration active.

2. Dispositif (1) selon la revendication 1, ***caractérisé par le fait que*** lesdites incisions (5) sont définies longitudinalement sur ledit tube (2).

3. Dispositif (1) selon la revendication 1, ***caractérisé par le fait que*** lesdites incisions (5) ont une longueur comprise entre 1 et 3 cm.

4. Dispositif (1) selon la revendication 1, ***caractérisé par le fait que*** ledit élément de compression (9) comprend des moyens d'engagement (22) avec au moins un premier élément de mouvement (16, 116) apte à amener lesdits moyens de retenue (3) au moins d'une configuration allongée à une configuration élargie.

5. Dispositif (1) selon la revendication 4, ***caractérisé par le fait que*** lesdits moyens d'engagement (22) comprennent au moins un premier filetage qui peut être engagé avec un second filetage (16a) défini sur ledit premier élément de mouvement (16).

6. Dispositif (1) selon la revendication 5, ***caractérisé par le fait que*** ledit premier filetage (22) est ménagé sur la surface intérieure dudit élément de compression (9).

7. Dispositif (1) selon l'une ou plusieurs des revendications précédentes, ***caractérisé par le fait que*** lesdits moyens de retenue (3) comprennent au moins un film de revêtement (23) associé extérieurement audit tube (2), au moins en correspondance avec lesdits éléments flexibles (4).

8. Dispositif (1) selon la revendication 7, ***caractérisé par le fait que*** ledit film de revêtement (23) est fait du même matériau que ledit tube (2).

9. Dispositif (1) selon l'une ou plusieurs des revendications précédentes, ***caractérisé par le fait qu*'il** comprend au moins un élément de retenue externe (12) associé audit tube (2).

10. Dispositif (1) selon l'une ou plusieurs des revendications précédentes, ***caractérisé par le fait qu*'il** comprend au moins un cathéter (21) d'alimentation entérale associé à l'orifice de sortie (2b) dudit tube (2).

11. Kit pour une alimentation entérale, ***caractérisé par le fait qu*'il** comprend au moins un dispositif (1) d'alimentation entérale selon l'une ou plusieurs des revendications 1 à 10, et au moins un premier élément de mouvement (16, 116) qui peut être inséré dans le tube d'alimentation (2) dudit dispositif (1) pour amener les moyens de retenue (3) du dispositif au moins jusqu'à une configuration élargie.

12. Kit selon la revendication 11, ***caractérisé par le fait que*** ledit dispositif (1) comprend au moins un élément de compression (9) selon la revendication 5 ou 6, **et *par le fait que*** ledit premier élément de mouvement (16) comprend un deuxième filetage (16a) apte à s'engager avec le premier filetage (2b) dudit élément de compression (9).

13. Kit selon la revendication 11, ***caratérisé par le fait que*** ledit dispositif (1) comprend au moins un élément de compression (9) selon la revendication 1, et ***par le fait que*** ledit premier élément de mouvement (116) comprend une extrémité (116a), destinée à sortir de l'orifice de sortie (2b) dudit tube (2) et qui peut être élargie pour s'engager avec ledit élément de compression (9).

14. Kit selon la revendication 13, ***caractérisé par le fait qu*'il** comprend au moins un deuxième élément de mouvement (18) qui peut être inséré dans ledit tube (2) pour amener lesdits moyens de retenue (3) dans la configuration allongée.

15. Kit selon la revendication 13 ou 14, ***caracterisé par le fait que*** ledit dispositif (1) comprend au moins un élément de compression (9) selon la revendication 1, et ***par le fait que*** ledit deuxième élément de mouvement (18) comprend une extrémité (18a) destinée à s'engager avec ledit élément de compression (9).

16. Kit selon l'une ou plusieurs des revendications 12 à 15, ***caractérisé par le fait qu*'il** comprend des moyens de coupe pour couper ledit tube (2).
